# FASCICULE DE BREVET EUROPEEN

(11) **EP 1 401 422 B1**
(45) Date de publication et mention de la délivrance du brevet: **04.04.2007**
(21) Numéro de dépôt: 02711967.6
(22) Date de dépôt: 16.01.2002
(51) Int. Cl.: A61K 31/195, A61K 31/185, A61K 33/20, A61P 31/00

(54) **COMPOSITION HALOGENEE, SON PROCEDE DE PREPARATION ET SES UTILISATIONS**
HALOGENHALTIGE ZUSAMMENSETZUNG, VERFAHREN ZU DEREN HERSTELLUNG UND DEREN VERWENDUNG
HALOGENATED COMPOSITION, METHOD FOR PREPARING SAME AND USES THEREOF

(30) Priorité: 23.01.2001 FR 0100862
(43) Date de publication de la demande: 31.03.2004
(73) Titulaire: Mainnemare, Arnaud, 76460 Neville (FR)
(72) Inventeur: Mainnemare, Arnaud, 76460 Neville (FR)
(74) Mandataire: Breese, Pierre
(86) Numéro de dépôt international: PCT/FR2002/000151
(87) Numéro de publication internationale: WO 2002/058692

(56) Documents cités:
- WO-A-00/74743
- DE-A- 4 041 703
- DE-A- 19 712 565
- DE-C- 19 816 102
- US-A- 3 998 945
- US-A- 4 035 483
- LIU Z X ET AL: "Negative chemotaxis in Cytophaga johnsonae." CANADIAN JOURNAL OF MICROBIOLOGY, (1996 MAY) 42 (5) 515-8., XP001030456
- MARTINDALE: "the complete drug reference" , PHARMACEUTICAL PRESS , LONDON XP002179622 32 page 1124, colonne 3
- DATABASE WPI Section Ch, Week 199540 Derwent Publications Ltd., London, GB; Class C03, AN 1995-308960 XP002179624 & JP 07 206609 A (SOGO YAKKO KK), 8 août 1995 (1995-08-08)
- DATABASE CA [en ligne] CHEMICAL ABSTRACTS SERVICE, COLUMBUS, OHIO, US; KIUCHI, KEIKO ET AL: "Microbicides containing nitrogen-containing halogen compounds" retrieved from STN Database accession no. 123:278710 XP002179623 & JP 07 206609 A (SOGO YATSUKO KK, JAPAN) 8 août 1995 (1995-08-08)
- KONTNY E. ET AL: "The mechanism of taurine chloramine inhibition of cytokine (Interleukin-6, Interleukin-8) production by rheumatoid arthriti fibroblast-like synoviocytes." ARTHRITIS AND RHEUMATISM, (2000) 43/10 (2169-2177)., XP001030454
- STIEF T W ET AL: "SINGLET OXYGEN INHIBITS AGONIST-INDUCED P-SELECTIN EXPRESSION AND FORMATION OF PLATELET AGGREGATES" CLINICAL AND APPLIED THROMBOSIS/HEMOSTASIS, HAGERSTOWN, MD, US, vol. 7, no. 3, 2001, pages 219-224, XP001030458 ISSN: 1076-0296
- CIRIOLO M.R. ET AL: '"The effects of hypolipidemic agents derived from procetofenic acid on the activity of superoxide dismutase and glutathione peroxidase and on malonyl dialdehyde production of rat liver."' ARZNEIMITTEL FORSCHUNG vol. 34, no. 4, 1984, pages 465 - 467, XP008046354

## Description

La présente invention concerne un mélange à base de composés halogènes pour le traitement d'infections virales, bactériennes, parasitaires, fongiques ou par des Agents Transmissibles Non Conventionnels (ATNC) mais aussi d'inflammations chroniques, progressives ou aiguës, pour des traitements immuno-modulateurs et/ou stimulateurs de la cicatrisation tissulaire ainsi qu'en rinçages pré- et/ou per- et/ou post- chirurgicaux. Le mélange de l'invention est tout particulièrement, utile comme antiseptique à usage local.

Le mélange de l'invention est basé sur l'association de deux types de substances actives; d'une part d'un antiseptique contenant au moins un hypochlorite de métal alcalin, et d'autre part, un dérivé N-halogéné d'une ou plusieurs molécule(s) de la famille des composés zwittérioniques et/ou de la famille des acides aminés.

L'Inventeur s'est intéressé aux caractéristiques de l'acide hypochloreux et des N-chloramines et à la compréhension des mécanismes mis en jeu lors de l'inflammation de façon à mettre au point la composition antiseptique de l'invention.

### 1) L'hypochlorite de métal alcalin.

L'hypochlorite de métal alcalin, et plus particulièrement l'hypochlorite de potassium et, surtout, de sodium (NaOCl), est utilisé depuis le début du 19^{ème} siècle pour ses propriétés antiseptiques. L'hypochlorite de métal alcalin est un sel de métal alcalin de l'acide hypochloreux (HOCl). Le titre de chlore actif, des solutions antiseptiques contenant de l'hypochlorite de sodium, est égal à la somme des concentrations de HOCl et de OCl⁻ (Bloomfield & miles 1979). La forme active de l'hypochlorite, l'acide hypochloreux est un très puissant agent oxydant qui a un rôle très important dans le système de défense des mammifères. Il est synthétisé principalement dans les Polynucléaires Neutrophiles et les monocytes (Wright et al. 1986) lors de la respiration oxydative par la réaction entre le peroxyde d'hydrogène et le chlore sous l'action de la myéloperoxydase. L'acide hypochloreux est très instable et réagit rapidement, surtout avec des amines primaires et secondaires pour donner des variétés de chloramines (Zgliczynski et al., 1971).

Dans le cytosol des polynucléaires, et spécialement les neutrophiles, un acide aminé, la taurine, est particulièrement abondant et, surtout, extrêmement réactif avec l'acide hypochloreux. Cette réaction donne la chloramine taurine. Cette chloramine est un oxydant beaucoup moins toxique et réactif que l'acide hypochloreux et c'est la plus stable de toutes les chloramines (Zgliczynski et al., 1971 & Marquez et Dunford, 1994). Ainsi, la taurine semble jouer un rôle protecteur important dans les milieux intra et extra-cellulaires en piégeant les molécules d'acide hypochloreux (Cantin, 1994, J. Marcinkiewicz et al., 1998). Cependant, du fait de sa longue demi-vie, les molécules de chloramine taurine peuvent être transportées à une grande distance du lieu où elles se sont formées et y exercer une action d'oxydation et/ou de chloration non négligeable (Zgliczynski et al., 1971).

Au pH physiologique (7,4), la réaction de la taurine et de HOCl se fait de manière spontanée et stoechiométrique (1/1 molécule) pour donner la N-monochloramine taurine (TauCl). A pH acide, cette réaction donne la N-monochloramine taurine et la N,N-dichloramine taurine (TauCl₂). Dans le milieu extracellulaire, les molécules qui réagissent le plus aisément avec l'acide hypochloreux sont la taurine et, surtout, les nitrites (NO₂⁻). Leurs concentrations y étant approximativement équivalentes, ce sont essentiellement les nitrites qui piège HOCl en formant des dérivés moins toxiques que TauCl. Par la formation de ces dérivés, les nitrites diminuent les propriétés bactéricides et immunologiques du HOCl (J. Marcinkiewicz et al., 2000). Dans le milieu intracellulaire des polynucléaires neutrophiles, la taurine, qui est très concentrée (20 mMol/l), s'impose pour piéger HOCl (J. Marcinkiewicz, 2000).

### 2) Les propriétés de l'hypochlorite de sodium, de l'acide hypochloreux et des N-chloramines.

### a) Capacité à dissoudre les tissus.

Il est également connu que l'hypochlorite de sodium, en solution aqueuse, est caustique ; c'est un agent non spécifique capable d'hydrolyser les tissus nécrotiques. Cette propriété est due à la présence d'hydroxyde de sodium NaOH. En plus de la concentration, la dissolution des tissus (surtout nécrosés) est fonction de la surface mise en contact avec NaOCl (Hand et al., 1978), du temps de contact et du volume de la solution de NaOCl utilisé (Thé et al.,1979).

Ainsi, même si une concentration de NaOCl inférieure à 0,5 % est insuffisante pour dissoudre totalement des tissus nécrosés, cette faible concentration est intéressante du fait de la réduction de sa toxicité. Cette faible capacité pour la dissolution des tissus nécrosés peut être compensée par un réchauffement de la solution d'hypochlorite à 37°C, même si, à cette température, la stabilité du NaOCl ne dépasse pas 24 heures.

### b) Stabilité de HOCl et de la TauCl en solution aqueuse.

- L'hypochlorite de sodium (NaOCl) :
   L'hypochlorite de sodium est une molécule très instable. A des concentrations inférieures à 5 g/l de chlore actif, sa stabilité ne dépasse pas 2 semaines. Plusieurs éléments influence cette stabilité:
      - La lumière : L'hypochlorite de sodium y est très sensible et doit en être protégé de par son mode de conditionnement.
      - La température : NaOCl est très sensible à des températures supérieures à 30°C.
      - La présence de métaux ou de matières organiques : Une solution d'hypochlorite formé de HOCl (NaOCl + H₂O ⇔ HOCl + NaOH) est consommée dans les interactions avec la matière organique. Pour qu'une solution d'hypochlorite soit efficace, elle doit pouvoir agir rapidement et être en excès par rapport à la quantité de matière organique.
      - Le pH: ainsi que cela a été expliqué dans le brevet EP 0 471 129 A1, une valeur du pH comprise entre 10 et 10,5 permet à l'hypochlorite de sodium d'avoir une excellente stabilité (supérieure à 24 mois) de son pouvoir oxydatif.
- La N-chloramine taurine (TauCl):
   Au pH physiologique (7.4) et à 37 °C, la TauCl est la plus stable des chloramines [la diminution de la capacité oxydative est inférieure à 5% par heure à 37°C] (Grisham MB, Jefferson MM, Melton DF, Thomas EL - J.Biol. Chem. 1984 ;259 :10404-13). Toutefois, comme le montre le brevet DE 40 41 703 A1, en solution aqueuse, la solubilité du sel de sodium de TauCl avec un Ph = 7-8 est excellente mais la stabilité de sa capacité oxydative est mauvaise : pour une valeur de Ph de 8,3 , elle chute d'environ 30% en quinze jours pour ensuite diminuer de 0,71% par jour (baisse de ~61% en 65 jours).

### c) Toxicité et viabilité cellulaire.

La toxicité est définie comme la perte significative des protéines intracellulaires. Cela se traduit par une perte de l'adhésion aux substrats et par une déformation cellulaire.

Les altérations de la viabilité cellulaire se mesurent par la diminution plus ou moins irréversible de l'activité mitochondriale et donc de la respiration cellulaire indispensable à la production énergétique.

La vulnérabilité des différents organismes cellulaires face à NaOCl et à TauCl est dépendante de nombreux facteurs:
- Du taux d'exposition de la surface cellulaire. Ainsi les systèmes qui mettent en oeuvres une organisation cellulaire (dans l'épithélium ou la plaque bactérienne, par exemple) sont moins vulnérables (les cellules en surface sont sacrifiées au profit des couches profondes) que les systèmes unicellulaires (procaryotes, cellules mobiles des mammifères, ou autres éléments unicellulaires).
- Du type de membrane qui protège les éléments intracellulaires et donc de leur niveau de perméabilité aux oxydants. Les plus efficaces sont les coques protéiques des virus.
- De la présence de membranes qui protègent les éléments intracellulaires clefs tels que l'ADN (noyau), la production d'énergie (mitochondries), les processus de sécrétion (appareil de Golgi), etc. Les procaryotes qui en sont dépourvus sont d'autant plus vulnérables.
- De la quantité intracellulaire d'anti-oxydants (comme par exemple le gluthatione, la taurine, les acides aminés, les groupes thiols, etc) qui est différente d'un type de cellule à un autre. Les moins riches en anti-oxydants sont les procaryotes.
- De la quantité extracellulaire d'anti-oxydants (comme la matière organique, les métaux, le sang, la matrice extracellulaire, etc.).
- Du flux liquide qui irrigue ces cellules et donc dilue la quantité d'oxydants.
- Du temps d'exposition
- Des conditions physico-chimiques locales (par exemples les propriétés tensioactives, oxydantes, olfactives ou gustatives, la stabilité, le pH, le pKa, la densité, la solubilité, la viscosité, la coloration, le coefficient de partage eau-ectanol).

Lors d'un traitement thérapeutique *in vivo,* les facteurs cités ci-dessus doivent être pris en compte lors de la détermination des dosages adéquats en agents actifs afin de pouvoir les adapter aux besoins réels de la situation clinique et au(x) objectif(s) recherché(s).
i) L'hypochlorite de sodium (NaOCl) ou l'acide hypochloreux (HOCl):
   - Sur des cellules de la lignée des macrophages RAW264.7 du rat. Pour [NaOCl] = 1 mmol/l, la viabilité cellulaire est très fortement affectée (irréversible) (Park E. et Al., 1997).
   - Sur des macrophages de souris, il y a une augmentation significative de la mortalité cellulaire pour toutes les concentrations d'HOCl supérieures à 0,125 mM. Cette cytotoxicité est complètement abolie par un excès de nitrite NO₂⁻ (NO₂⁻ seuls ne présente pas d'activité cytotoxique) (Marcinkiewicz. J. et Al., 2000).
   - *In vitro* sur les macrophages, les fibroblastes, les kératinocytes humains:
      - Pour [NaOCl] = 13,433 mmol/l, la toxicité de NaOCl est si rapide qu'elle n'a pas le temps d'être neutralisée par des antioxydants (à des concentrations compatibles physiologiquement).
      - Pour [NaOCl] > 6,7165 mmol/1, NaOCl présente une grande toxicité
      - Pour [NaOCl] < 3,358 mmol/l, la toxicité peut être maîtrisée par un ajout d'antioxydant.
      - Pour [NaOCl] < 1,679 mmol/l, la toxicité est très faible en présence d'antioxydants (Hidalgo E. & Dominguez C., 2000).
   - La perte d'adhérence des macrophages en présence de HOCl a été étudiée. Pour [NaOCl] = 1,0075 mmol/l, après deux heures de contact in vitro, 95% des cellules sont vivantes mais seulement 40% conserve leur adhérence au substrat.
   - Sur des cellules endothéliales humaines in vitro (Pullar JM et Al. en 1999).
      . Pour [HOCl] ≤ 25 µmol/l, HOCl n'est pas toxique.
      . Pour [HOCl] > 25 µmol/l, il est observé une augmentation progressive de la toxicité cellulaire qui est également dépendante du temps d'exposition.
      . Pour [HOC1] = 50 µmol/l il y a des contractions cellulaires, leurs formes s'arrondissent dans les 10 mn et les cellules commencent à se détacher au bout d'une heure avec une majorité de cellules détachées après 3 heures.
   - Sur des fibroblastes humains in vitro.
      . Pour [NaOCl] ≥ 1,0075 mmol/l (observée pendant 24 heures après une exposition de 15 minutes) la viabilité cellulaire est affectée
      . Pour [NaOCl] = 16,791 mmol/l la mortalité cellulaire est totale.
      . Pour 67,165 µmol/l < [NaOCl] < 671,655 µmol/l, 100% des cellules exposées sont vivantes.
      . Pour [NaOCl] < 671,655 µmol/l, et en présence de 2% de FCS, la viabilité des fibroblastes qui sont exposées pendant 24 heures n'est pas affectée et il est même observé une stimulation de la croissance et de la prolifération des fibroblastes qui augmente avec la diminution de la [NaOCl] avec une efficacité maximale à 33,582 µmol/l [Hidalgo E. et Dominguez C., Life Sci. 2000 Aug 4;67(11):1331-44].
      . Pour [HOCl] < 50 µmol/l n'affecte pas la viabilité des fibroblastes cutanés humains in vitro. A ces concentrations HOCl ne provoque pas d'apoptose cellulaire (Vile G.F. et Al., 2000).
ii) Effets de la N-chloramine taurine [TauCl] sur la viabilité cellulaire:
   - Sur les gliocytes (glioma cells) C6 du rat in vitro. Pour [TauCl] = 0~2 mmol/l, la viabilité cellulaire n'ai pas affectée (Liu Y. et Al., 1999).
   - Sur les fibroblastes cutanés humains in vitro
      Pour [TauCl] ≤ 100 µmol/l il n'y a pas de cytotoxicité. A ces concentrations, TauCl ne provoque pas d'apoptose cellulaire (Vile G.F. et Al., 2000).
   - Sur des synoviocytes humains (lignée des fibroblastes), en présence de hautes concentrations de TauCl (400 - 500 µmol/l), les cellules changent de morphologie (~30 - 50% des cellules prennent une forme arrondie et sont détachées de la surface plastique) bien que la viabilité soit conservée (≥ 95%) (Kontny E. et Al., 1999).
   - Sur les lymphocytes T de la sourie
      . Pour [TauCl] = 30-300 µmol/l, TauCl n'affecte pas la viabilité cellulaire (établie au niveau l'activité mitochondriale).
      . A 300 µmol/l, TauCl est cytotoxique pour les lymphocytes du type DO-10-11 (Marcinkiewicz J. et Al., 1998)
   - Sur les cellules dendritiques de la sourie, qui sont incubées 24 heures avec TauCl :
      - Pour 0,05 mmol/l < [TauCl] < 0,5 mmol/l, l'activité mitochondriale et donc la viabilité de ces cellules n'est pas affectée.
      - Pour [TauCl] > 0,5 mmol/l et avec des incubations de 24 heures, il est observé une diminution significative de la viabilité cellulaire (Marcinkiewicz J., et Al., 1999)
   - Sur les macrophages ou sur cellules de la lignée des macrophages, Pour [TauCl] .= 0,05~0,6 mmol/l, la viabilité cellulaire n'est pas affectée. Elle l'est à partir de 1 mmol/l. (Marcinkiewicz J., et Al., 1995)

### d) Assimilation cellulaire de l'HOCl exogène et de la chloramine taurine exogène.

HOCl est un agent oxydatif lipophile et possède de ce fait une grande facilité pour traverser les membranes cellulaires. Celle-ci est très rapide (~ 80% de HOCl est assimilée en dix minutes par des fibroblastes humains) (Vile G.F. et Al., 2000). Sur des cellules endothéliales en culture in vitro, avec [HOCl] = 35 µmol/l, 50% des molécules de HOCl sont consommées en ½ minute, la totalité en ~ 15 minutes avec la grande majorité de celles-ci en dix minutes (Pullar J.M. et Al., Am J Physiol. 1999 Oct;277(4 Pt 2):H1505-12).

TauCl est assimilé des systèmes de transport membranaire spécifiques. Ainsi pour les cellules RAW264.7 du rat au repos, la valeur in vitro du Kₘ et de la Vₘₐₓ sont respectivement de 23,3 µmol/l et de 51,3 pmol/min/10⁶ cellules (Kₘ = 28,1 µM et Vₘₐₓ = 90,9 pmol/min/10⁶ cellules pour la taurine). Pour des macrophages stimulés par des LPS, les valeurs in vitro sont de Kₘ = 45,9 µmol/l et Vₘₐₓ = 82,6 pmol/min/10⁶ cellules pour TauCl et de Kₘ = 17,3 µM et Vₘₐₓ = 116,3 pmol/min/10⁶ cellules pour la taurine.

Les systèmes de transport membranaire de la TauCl et de la taurine sont indépendants et les deux systèmes d'assimilation sont actif et sont dépendants de la température, du Na⁺ et de l'énergie.

La biodistribution de la TauCl et de la taurine par le sang se traduit par une assimilation rapide au niveau des poumons, de foie, de la rate, de l'estomac, de l'intestin et les reins. Il est noté une grande assimilation de la taurine et de la TauCl au niveau des cellules présentes sur les lésions inflammatoires (rapport inflammation/sang sont respectivement de 6,43 et de 4,84) (Kim C., et Al., 1998). D'autres données ont montré une assimilation rapide par les reins, le foie, la rate et la moelle osseuse tandis que cette assimilation est lente au niveau du coeur et des muscles (Huxtable RJ, J. Nutr. 1981 ; 111 :1275-86).

### e) Les propriétés antiseptiques.

L'hypochlorite de sodium est un très puissant et très efficace agent anti-bactérien, antiviral et antifongique (Shih & al., 1970; Bloomfield & Miles, 1979 ; Harrison & Hand, 1980). Un effet bactéricide, sur des bactéries gram- et gram+, a été observé, *in vitro,* jusqu'à des concentrations de NaOCl de 3,36 mmol/l (0,025%) (Heggers JP et al. 1991). La concentration minimale pour détruire le virus HIV est de 19,062 mmol/l (0.1%) de chlore actif.

Des études sur la chloramine taurine montrent que celle-ci affecte significativement la viabilité de E. *Coli* mais seulement à un pH acide, ce qui indique que c'est la dichloramine et non la monochloramine taurine qui possède une activité bactéricide (J. Marcinkiewicz, 2000). La N-monochloramine taurine aurait donc une activité antiseptique très réduite, voire nulle.

### 3) L'inflammation.

L'inflammation est une réaction de défense vis à vis de tout type d'agression. L'agresseur est détecté par des cellules sentinelles, comme les macrophages et les cellules dendritiques (CD). En réponse, un processus va se déclencher qui aura pour conséquence l'initialisation du système immunitaire, via la génération et la libération de médiateurs (J. Marcinkiewicz et al., 1999) . Ces médiateurs vont, donc, déclencher une série de réactions en chaîne dans le but d'activer le système immunitaire, d'adapter sa réponse au type d'agression et de favoriser son intervention. Lorsque l'agresseur aura été éliminé, un processus de cicatrisation/réparation sera mis en place.

Deux types d'immunité sont reconnues: l'innée (naturel) et l'acquise (adaptative).

Le composant cellulaire de l'immunité innée (naturelle) est composé des monocytes (phagocytes mononucléaires), des polynucléaires neutrophiles (PNN) et des cellules natural killer (NK). Ces cellules utilisent la cascade du complément comme un mécanisme de protéines effectrices primaires, aussi bien que des protéines de reconnaissances variées comme la protéine C réactive et la protéine amyloïde, parmi d'autres. Ces protéines sont capables de se lier aux structures carbohydrates présentes sur les bactéries mais pas sur les cellules eucaryotiques. Les PNN font parties de la première ligne de défense et sont en étroite coopération avec les macrophages, qui sont des cellules effectrices majeures du système immunitaire ; les PNN sont responsables de la défense non spécifique dans l'inflammation aiguë et les macrophages ont le même rôle dans l'inflammation aiguë et chronique (J. Marcinkiewicz et al., 1994).

L'immunité acquise (adaptative) implique plusieurs sous types de lymphocytes et utilise les anticorps comme protéines effectrices. Les récepteurs de cellules T et les anticorps sont des molécules de reconnaissances. Les lymphocytes B reconnaissent les carbohydrates, les protéines et quelques structures chimiques relativement simples tandis que les lymphocytes T reconnaissent seulement les peptides. Les cellules dendritiques y jouent un rôle non négligeable. Sous l'action de médiateurs inflammatoires, les DC effectuent une migration des tissus non lymphoïdes vers les organes lymphoïdes où elles vont perdre leur habilité à la capture des antigènes et acquérir une capacité croissante pour stimuler les lymphocytes T (J. Marcinkiewicz et al., 1994).

### 4) Les médiateurs de l'inflammation.

Les cytokines sont les plus importantes molécules messagers intercellulaires du système immunitaire (Megarbane B. et al., 1998). Générées par des cellules immunitaires activées, elles engendrent des activités biologiques particulières, après liaison avec un récepteur sur la cellule cible de la réponse et cela, soit de manière autocrine, soit de manière paracrine. Les macrophages et les lymphocytes T sont les principales cellules productrices de cytokines, toutefois, beaucoup d'autres cellules sont capables également de les générer et de les libérer. Les cytokines sont de véritables régulateurs de la réponse immunitaire humorale et cellulaire. Les cytokines travaillent en concert, et la balance entre leurs activités est cruciale pour la régulation du système immunitaire. La plus connue est la compétition entre les cytokines des lymphocytes T TH1 (IL-2, INF-γ, TNF-β et IL-12) et TH2 (IL-4, IL-5, IL-10 et. IL-13).

Les lymphocytes TH1 sont impliqués dans l'immunité cellulaire et elles sont responsables des activités cytotoxiques des macrophages, des lymphocytes T cytotoxiques (CTL) aussi bien que les cellules « natural killer » (NK).

Les lymphocytes TH2 sont associés avec la réponse humorale. Ainsi, IL-10, une cytokine de type TH2, inhibe puissamment les fonctions effectives des macrophages et des cellules TH1 (J. Marcinkiewicz, 1997).

Les fonctions régulatrices des cytokines peuvent être étendues à la sélection des isotypes des immunoglobulines lors de la réponse humorale. Ainsi, des inhibitions sélectives de cytokines résultent en une modulation de la réponse immunitaire.

Les eicosanoïdes (prostaglandines et leukotriènes) et l'oxyde nitrique (NO), produits par les macrophages activés, ont un grand impact sur la régulation de la production des cytokines. Les eicosanoïdes (leukotriènes, prostaglandines) ne sont pas trouvés préformés dans les tissus. Ils sont générés à partir de l'acide arachidonique qui est lui-même dérivé des phospholipides des membranes cellulaires.

Les prostaglandines (PG) sont catalysées par la cyclooxygénase (COX) qui convertie l'acide arachidonique en cycliques endopéroxydes. Il existe une forme constitutive (COX1) et une forme induite (COX2) de la COX. La dernière est activée dans les cellules inflammatoires par des agents pro-inflammatoires. Dans les macrophages, cela mène principalement à la synthèse des prostaglandines E₂ (PGE₂) et des prostacyclines I₂ (PGI₂) et dans les mastocytes à la synthèse des prostaglandines D₂.

Les prostaglandines (particulièrement PGE₂) et les leukotriènes (particulièrement LTB₄) modifient les réponses immunitaires et un équilibre entre les effets de ces eicosanoïdes variés permet un fonctionnement harmonieux du système immunitaire.

L'oxyde nitrique (NO) est synthétisé à partir de la L-arginine par les deux formes de l'oxyde nitrique synthétase, la forme constitutive, calcium dépendante (cNOS) et la forme induite, calcium indépendante (iNOS). La cNOS est responsable de la synthèse de la forme basale de l'oxyde nitrique à la fois dans l'endothélium et dans le système nerveux. La iNOS est trouvée dans une variété de cellules, incluant les macrophages, les neutrophiles et les hépatocytes. La génération de l'oxyde nitrique joue un rôle important dans la cytotoxicité des macrophages et de leur habilité à détruire les organismes envahisseurs et donc, dans la défense non spécifique de l'hôte contre de nombreux pathogènes et contre des cellules tumorales.

Les caractéristiques de ces médiateurs de l'inflammation ont été décrites dans l'art antérieur (Knight JA, 2000 ; Marcinkiewicz J., 1997 ; Marcinkiewicz J., 1997 ; Megarbane B, 1998).

### 5) Influence de l'acide hypochloreux et de la N-chloramine taurine au niveau du site inflammatoire.

- Sur les bactéries.
   Après stimulation par des bactéries Gram+ (Staphylococcus aureus, S. epidermidis, Escherichia coli) non chlorées, les macrophages péritonéaux du rat libèrent des fortes concentrations d'oxyde nitrique, de TNF-α, et d'IL-6. Les bactéries chlorées par HOCl perdent leur habilité à induire l'oxyde nitrique et le TNF-α, tandis que la production d'IL-6 et la phagocytose ne sont pas affectées (J. Marcinkiewicz et al., 1994).
- Sur l'endothélium.
   HOCl augmente la perméabilité de l'endothélium et favorise l'adhérence des leukocytes à l'endothélium de la microcirculation. La TauCl atténue l'augmentation de la perméabilité de l'endothélium due à l'action des PNN. La taurine seule n'a aucun effet (Tatsumi et Flies, 1994).
- Sur la croissance cellulaire.
   Les effets de l'acide hypochloreux sur des cellules endothéliales des veines ombilicales humaines en culture ont été étudiés et il a été montré que de très faibles concentrations (5 nmol de HOCl pour 1,2x10⁵ cellules) ne provoquent pas la mort cellulaire, mais un arrêt transitoire de la croissance (Vissers MC, Pullar JM, Hampton MB, 1999). Il a aussi été montré que de faibles doses de HOCl et de chloramines physiologiques mènent à une inhibition de la synthèse d'ADN et de la division cellulaire de fibroblastes cutanés en culture (Vile GF, Rothwell LA, Kettle AJ, 2000).
- Sur les protéines non libres (comme le collagène, etc.).
   HOCl est un très puissant oxydant. Il modifie les protéines par chloration et les rend plus vulnérable à la dégradation par les endopeptidases. Cette dégradation contribue à la destruction des tissus environnants le site inflammatoire. TauCl, qui est un oxydant beaucoup moins puissant, semble peu responsable de ces dommages tissulaires.
- Sur la collagènase.
   TauCl exerce une inhibition/inactivation directe de la collagènase tandis qu'elle n'a aucun effet sur la susceptibilité protéolytique du collagène lui-même. En comparaison, les N-monochloramines leucines et alanine et HOCl n'ont aucun effet inhibiteur sur la collagènase; au contraire, les N-monochloramine leucine et alanine potentialisent la susceptibilité protéolytique du collagène (Joanna M. S. Davies et al., 1994).
- Sur les protéines libres (ovalbumine, enzymes bactériennes ou autres, etc.).
   La chloration des protéines libres augmente leur sensibilité immunitaire, probablement en facilitant le traitement et la présentation de ces protéines par les « cellules présentatrices des antigènes » (APC), principalement les macrophages et les cellules dendritiques (DC). Cette chloration est dix fois plus importante pour HOCl que pour TauCl, mais, in vivo, c'est la TauCl, grâce à sa stabilité, qui en est principalement responsable (J. Marcinkiewicz et al., 1999)
- Sur les cellules dendritiques (DC).
   La N-monochloramine taurine (TauCl), préincubée avec des DC du rat pendant 2 heures, possède une action inhibitrice qui est varie en fonction de la concentration de TauCl. Pour [TauCl] = 0,5 mmol/l, TauCl inhibe presque complètement les sécrétions, par les DC, de l'oxyde nitrique, de PGE₂, des agents oxygénés réactifs (ROS) générés par la respiration oxydative, et des cytokines TNF-α, IL-6, IL-10 et IL-12. Est également inhibé l'expression, induite par les lipopolyssacharides, des MHC classe II et des molécules B7-2. A cette concentration, TauCl peut être toxique pour les DC si celles ci sont exposées pendant une longue durée. Pour [TauCl] = 0,25 mmol/l, TauCl à une action plus sélective. Elle inhibe la production d'IL-12, IL-10, de PGE₂, et de l'oxyde nitrique, mais pas celle de TNF-α et des ROS. De plus, l'exposition des DC à TauCl semble favoriser le développement d'une réponse lymphocytaire TH1 et une diminution de l'activité des Th2 (J. Marcinkiewicz et al., 1999).
- Sur les lymphocytes T.
   TauCl inhibe la libération de IL-2, IL-6 par des lymphocytes T , lorsque ceux-ci sont préincubés avec une concentration de TauCl de 0.1 à 0.3 mmol/l et stimulées par un mitogène, un antigène ou un complexe ovalbumine-APC (J. Marcinkiewicz et al., 1998).
- Sur les phagocytes.
   Les antigènes chlorés par HOCl et les antigènes qui sont en présence de TauCl, ne stimulent pas la production de médiateurs inflammatoires par les phagocytes qui les ont phagocytés.
- Sur les macrophages.
   Les chloramines telles que taurine monochloramine, taurine dichloramine, N-monochloroéthanolamine et N-dichlorophosphoéthanolamine aussi bien que NaOCl (hypochlorite de sodium), tous inhibent la libération de l'oxyde nitrique, et cela d'une manière dose-dépendante. La sérine chloramine est active immédiatement après sa préparation (0,3 mmol/l de SerCl, inhibition de la génération de l'oxyde nitrique de 85%). Elle perd son activité inhibitrice après 24 heures au repos dans une solution (inhibition de 22%). La demi-vie de l'activité de la SerCl est donc courte. La N-monochloramine taurine inhibe la génération de l'oxyde nitrique de 98 % pour 0.6 mmol/l et de 8 à 22 % (en fonction du type de cellules) pour 0,1 mmol/l de TauCl. Cette inhibition s'effectue au niveau de la transcription de gène de iNOS. La taurine seule est sans effet sur cette transcription (J. Marcinkiewicz. Et al., 1995). HOCl (probablement grâce à TauCl) et TauCl inhibe l'expression post transcriptionnelle (retard de 4 heures de la cinétique de l'expression de l'ARNm) de COX2 (et donc la production de PGE₂) et diminue la vitesse de transcription de TNF-α; d'une manière dose-dépendante avec une IC₅₀ de 0.4 mmol/l (Quinn M.R. et al., 1996). TauCl inhibe l'expression de COX2 par les macrophages, quel que soit leur niveau de stimulation par INF-γ. TauCl inhibe la production de TNF-α, d'IL-6 et l'expression de iNOS uniquement par les macrophages stimulés par INF-γ. Elle n'a aucune action sur la production d'IL-1α, quel que soit le niveau de stimulation. La taurine native seule est sans effet sur tous ces médiateurs. Les lipoprotéines du plasma, qui ont été oxydées par HOCl, ont l'habilité de diminuer la synthèse, par les macrophages, de l'ARNm de iNOS et donc d'inhiber la synthèse de l'oxyde nitrique. Elles contribuent au développement des lésions athérosclérotiques (Moeslinger T. et al., 2000)
- Sur les PN neutrophiles.
   TauCl inhibe la production de l'oxyde nitrique, de la prostaglandine E₂, de l'interleukine-6, du TNF-α et cela d'une manière dose-dépendante. La taurine native, seule, est sans effet. Grâce à des mesures de la chimiluminescence dépendante du luminol (LCL), il a été confirmé ou montré que (J. Marcinkiewicz et al., 1998 & 2000):
- Les ROS sont réduites à la fois par la taurine et par la N-chloramine taurine. Toutefois, la taurine affecte LCL à hautes concentrations et son action est beaucoup moins étendue que celle de TauCl.
- L'acide hypochloreux exerce une inhibition dose-dépendante, de type rétroactive, sur l'activité de la myélopéroxydase. TauCl et HOCl ont un effet similaire sur l'activité de la myéloperoxydase, in vitro, quand ces agents sont additionnés à de la myéloperoxydase qui été extraite des neutrophiles. L'acide hypochloreux exerce une inhibition dose-dépendante sur la production du peroxyde d'hydrogène. Cette inhibition (pour HOCl = 0.25 mmol/l) est stoppée par la taurine (0.5 mmol/l) ou les nitrites (0.25 mmol/l). TauCl n'a pas d'effet sur cette production.
- Une diminution de la chimiluminescence, dose-dépendante, est observée avec HOCl ou TauCl, TauCl (IC₅₀= 0.55 mmol/l) étant moins efficace que HOCl (IC₅₀= 0.1 mmol/l).
- La taurine et la TauCl inhibent la production de l'anion superoxyde (O₂⁻) par des neutrophiles stimulés.
- Cette inhibition de la production de O₂⁻ se fait par un mécanisme distinct de la réaction de la taurine avec l'acide hypochloreux (pour donner TauCl) car la combinaison de la taurine (ou de la TauCl) avec un inhibiteur spécifique de la myéloperoxydase a un effet synergique. Ce mécanisme d'action reste à élucider.
   Toutefois, la taurine affecte la LCL à hautes concentrations et son action est beaucoup moins étendue que celle de TauCl (Marcinkiewicz J et Al., 1998).
- Sur les PN éosinophiles.
   Les leucotriènes sulfidopeptides LTC₄ sulfoxides et 6-trans-LTB₄ sont inactivés, uniquement dans le milieu extracellulaire, par HOCl (Owen W. F. et al., 1987).
- Sur les gliocytes C6 du Rat.
   Dans le système nerveux central, TauCl inhibe la production par les gliocytes activés, de la protéine-1 chémoattractante des monocytes (MCP-1) et la protéine-2 inflammatoire du macrophage (MIP-2) d'une manière dose-dépendante et au travers du système post-transcriptionnel (Liu Y , Schuller-levis G, Quinn MR., 1999). TauCl inhibe, également, l'expression transcriptionnelle du gène iNOS, et donc la production de l'oxyde nitrique. Elle inhibe également l'expression des protéines COX-2 et donc la production de PGE₂, par un mécanisme post-transcriptionnel (Liu Y et al., 1998).
- Sur les fibroblastes.
   Selon E. Kontny et al., 1999; la TauCl inhibe la prolifération des synoviocytes (semblables aux fibroblastes) chez des patients atteints de d'arthrite rhumatoïde. En 2000, sur le même type de cellules et pour la même pathologie, ils ont montré que TauCl possède l'habilité à diminuer l'activité des facteurs de transcription majeurs de IL-6 (valeur IC₅₀ ~ 225 µmol/l) et de IL-8 (valeur IC₅₀ ~ 450 µmol/l) et donc de réduire la transcription de ces gènes d'une manière dose dépendante. TauCl diminue donc l'action proinflammatoire due à IL-6 et temporairement la faculté de ces cellules immunitaires à migrer dans le site inflammatoire (inhibition de IL-8). Pour IL-6, cette inhibition est indépendante du médiateur proinflammatoire qui a stimulé le fibroblaste (que ce soit TNF-α ou IL-1β ou IL-17). Pour IL-8, cette inhibition s'effectue pour les stimulations faites par TNF-α ou IL-1β mais pas pour celles dues à IL-17. Cela indique que les voies suivies par les signaux déclenchant la transduction sont différentes entre TNF-α / IL-1β et IL-17 (Kontny E, 1999).
   Pour une stimulation de synoviocytes humains atteints de RA par IL-1β, l'inhibition de la transduction de IL-6 et de IL-8 par TauCl s'effectue au niveau de deux de leurs facteurs de transcription: NF-κB et AP-1.
   TauCl inhibe à la fois la prolifération spontanée et celle déclenchée par bFGF des synoviocytes de patients atteints de RA.
   De faibles doses de HOCl et de chloramines physiologiques (NH₂Cl, TauCl et les acides α-aminés N-chlorés) mènent à une inhibition à la fois de la synthèse d'ADN et de la division cellulaire des fibroblastes cutanés humains en culture (Glenn F. Vile et al., 2000).
- Sur les facteurs de transcription NF-κB et AP-1.
   L'expression de tous les gènes dont la transcription dépend essentiellement de l'action de NF-κB a de forte chance d'être affecté par l'action de TauCl. Pour une stimulation de synoviocytes humains atteints de RA par IL-1β, l'inhibition de la transduction d'IL-6 et d'IL-8 par TauCl s'effectue au niveau de deux de leurs facteurs de transcription: NF-κB et AP-1 et cela en réduisant la faculté de liaison de ces facteurs avec l'ADN d'IL-6 et de IL-8. La transcription d'IL-6 est sous le contrôle de NF-κB, tandis que pour IL-8, l'action de AP-1 et de NF-κB sont nécessaire. A 250 µM, TauCl réduit sélectivement le collage de NF-κB et donc la transcription d' IL-6 sans affecter le collage de AP-1 et donc la transcription de IL-8. A 500 µM de TauCl, l'activité de collage de NF-κB et de AP-1 sont toutes les deux diminuée, d'où réduction de la transcription de IL-6 et de IL-8 (Kontny E., 2000).
   Cette régulation s'effectue par un mécanisme d'oxydoréduction (redox) [(Sen C.K., Packer L., Fased J. 1996;10 :709-20), (Li N. & Karin M., Fased J. 1999;13 :1137-43), (Kunsch C. & Medford R.M., Circ Res. 1999 Oct 15;85(8):753-66.)]. Kontny et Al. 2000, ont émis l'hypothèse que TauCl, qui un oxydant faible, pourrait interférer avec le statut cellulaire oxydo-réducteur de ces facteurs de transcription. Ces auteurs suggèrent en conclusion que NF-κB pourrait représenter un puissant facteur physiologique anti-inflammatoire.
- Sur le complément.
   Le composant C₅ du complément humain peut être activé par des oxydants tels que les radicaux hydroxyl, l'hypochlorite et les chloramines (TauCl, et surtout NH₂Cl). L'activation repose sur un changement structural de C₅, qui est atteint sans clivage du peptide, et qui est induite par l'oxydation des résidus méthionine dans la protéine de C₅. Les changements mènent à l'expression du site de liaison C₆ qui, normalement, est formé après un clivage spécifique de C₅ dans C₅ₐ et C_{5b}, par une des deux convertases C₃/C₅. Dans la mesure où le produit de l'oxydation de C₅ est semblable à C_{5B}, il est capable de débuter l'assemblage du complexe membrano-lytique C₅₋₉.
   Les fragments chémotactiques ne sont pas directement générés, mais les C₅ (semblables aux C_{5b}) activés sont rapidement attaqués par des enzymes tel que la Kallikrein, ce qui produit des fragments semblables à C₅ₐ et ayant une activité chémotactique. Il est probable que le complexe C₅₆₇ formé avec C₅ (semblable à C_{5b}) soit aussi chémotactique, tout comme le complexe C_{5b67}. Le complexe C_{5b}-9 est connu pour stimuler, à concentrations non toxiques, les PNN. Le complexe correspondant C₅₋₉ formé avec le C₅ (semblable au C_{5b}) a très vraisemblablement le même effet. Ainsi, cela peut mener à un cercle vicieux qui augmente les lésions tissulaires (Vogt Walther, 1996).

### 6) Description détaillée de l'invention.

Ainsi sur la base des observations précédentes, il est maintenant établi que NaOCl, qui possède une action bactéricide prononcée, contribue, dans l'inflammation, à l'accélération du passage à la phase de détersion des masses nécrotiques et purulentes, stimule l'immunité locale et active le processus de réparation (Lelianov AD et al., 1991). Des propriétés qui ont pour origine l'association des deux composés de l'hypochlorite de sodium, le radical hydroxyl (pour l'assainissement) et l'acide hypochloreux, et surtout, les dérivés chlorés de ce dernier.

En conséquence, la présente , invention a pour objet l'utilisation d'un mélange d' au moins un hypochlorite de métal alcalin et d'au moins une N-haloamine taurine pour la préparation d'un médicament destiné, chez l'homme ou l'animal, au traitement d'infections virales et/ou bactériennes et/ou parasitaires et/ou fongiques et/ou par des agents transmissibles non conventionnels (ATNC); et/ou d'inflammations chroniques, progressives ou aiguës; et/ou pour des traitements immuno-modulateurs et/ou stimulateurs de la cicatrisation tissulaire ; ainsi qu'aux rinçages pré et/ou per et/ou post chirurgicaux, ledit médicament n'exerçant pas de stimulation de l'activité de la myéloperoxydase.

Avantageusement, l'hypochlorite de métal alcalin est l'hypochlorite de sodium, et la N-haloamine taurine est la N-chloramine taurine.

Le mélange de l'invention est remarquable en ce qu'il présente des propriétés antiseptiques à très large spectre, des propriétés anti-inflammatoires, des propriétés de modulation de l'immunité et des propriétés de stimulation de la cicatrisation tissulaire ; sans stimuler l'activité de la myélopéroxydase.

L'hypochlorite de métal alacalin et la N-haloamine taurine sont associés dans le mélange selon l'invention avec un excipient, comme l'eau purifiée, conforme pour un usage thérapeutique. Il s'agit de préférence d'eau purifiée osmosée (isotonique). Cet excipient peut contenir des agents divers, pharmaceutiquement compatibles avec l'hypochlorite de métal alacalin et la N-haloamine taurine, permettant de modifier certaines propriétés physico-chimiques, (exemples: les propriétés tensioactives, oxydantes, olfactives ou gustatives, la stabilité, le pH, le pKa, la densité, la solubilité, la viscosité, la coloration, le coefficient de partage eau-ectanol), du mélange de l'invention. Le mélange de l'invention peut aussi comprendre des anti-oxydants et/ou des acides aminés qui auront un effet de dilution en neutralisant quelques molécules l'hypochlorite de métal alcalin. Ces anti-oxydants, ces acides aminés et leurs dérivés halogénés auront une action pharmaceutique soit neutre, soit dirigée vers les effets thérapeutiques recherchés et n'exerceront pas de stimulation directe, en présence des agents actifs qui entrent dans le médicament de l'invention, de l'activité de la myéloperoxydase.

Le mélange selon l'invention pourra être commercialisée sous une forme à préparer avant l'emploi consistant à mélanger le(s) hypochlorite (s) de métal alcalin avec le(s) N-haloamine(s) taurine et un ou plusieurs excipients. Cette forme de présentation pourra être envisagée s'il est nécessaire de garantir une meilleure stabilité dans le temps de la composition et des produits la constituant. Toutefois, même sous une présentation où les produits la constituant seront associés, Le mélange de l'invention pourra être commercialisé accompagné d'un excipient, tel que l'eau purifiée, conforme pour un usage thérapeutique. I1 s'agit de préférence de l'eau purifiée osmosée (isotonique). Cet excipient pourra en outre contenir des agents divers, pharmaceutiquement compatibles avec l'ensemble des molécules du mélange thérapeutique final, dans le but de modifier certaines propriétés physico-chimiques de la composition (exemples: les propriétés tensioactives, oxydantes, olfactives ou gustatives, la stabilité, le pH, le pKa, la densité, la solubilité, la viscosité, la coloration, le coefficient de partage eau-ectanol) par l'ajout d'un(des) agent(s) adéquat(s).

le mélange pourra également être préparé avant son administration au patient en mélangeant les constituants décrits ci-dessous .
- (i) au moins un hypochlorite de métal alcalin, et
- (ii) au moins une N-haloamine taurine.

Avantageusement, l'hypochlorite de métal alcalin est l'hypochlorite de sodium, et la N-haloamine taurine est la N-chloramine taurine.

Le(s)dit(s) hypochlorite(s) de métal alcalin sont avantageusement présentés sous la forme d'une solution liquide ou semi-liquide comme un gel, avantageusement dans un excipient comme décrit ci-dessous. Cette solution d'hypochlorite pourra être stabilisé selon le procédé décrit dans le brevet EP 0 471 129 A1 par un agent de réglage du pH afin d'obtenir un pH compris entre 10 et 10,5 tout en respectant la viabilité cellulaire.

Le(s)dit(s) N-haloamine(s) taurine sont avantageusement présentés sous la forme d'une solution liquide ou semi-liquide comme un gel, avantageusement dans un excipient comme décrit ci-dessous.

Avantageusement, le mélange composition de l'invention sera préparé en mélangeant les deux solutions ci-dessus avec un au moins excipient comme de l'eau purifiée, conforme pour un usage thérapeutique. Il s'agira de préférence d'eau purifiée osmosée (isotonique). Cet excipient pourra en outre contenir des agents divers, pharmaceutiquement compatibles avec l'ensemble des molécules du mélange final, dans le but de modifier certaines propriétés physico-chimiques de la composition, (exemples: les propriétés tensioactives, oxydantes, olfactives ou gustatives, la stabilité, le pH, le pKa, la densité, la solubilité, la viscosité, la coloration, le coefficient de partage eau-ectanol) par l'ajout de(s) l'agent(s) adéquat(s).

Une variante au procédé ci-dessus consiste à mélanger :
- (i) au moins un hypochlorite de métal alcalin comme défini ci-dessus, se présentant sous la forme d'une solution liquide ou semi-liquide comme un gel, avantageusement dans un excipient comme décrit ci-dessus, et
- (iii) au moins un acide aminé choisi parmi la taurine, désigné ci-après également « Zw/Aam », se présentant sous la forme d'une solution liquide ou semi-liquide comme un gel, avantageusement dans un excipient comme décrit ci-dessus,
de façon à obtenir une association d'au moins un hypochlorite de métal alcalin et d'au moins une haloamine, le tout en quantité thérapeutique suffisante pour inhiber la myéloperoxydase.

Ce mélange est de préférence réalisé avec un excipient comme défini ci-dessus.

Dans ce mode de réalisation, les dérivés formés seront N-chlorés, et plus particulièrement des N-chloramines.

Le titre en hypochlorite de la première solution active principale devra tenir compte de la stoechiométrie et du taux de réactivité de la réaction entre l'acide hypochloreux et les molécules Zw/Aam. Pour le cas ou la réaction ne serait pas complète, les molécules Zw/Aam qui subsisteraient, ne devront pas être des stimulateurs, en présence des agents actifs qui entrent dans la composition de l'invention, de l'activité de la myélopéroxydase.

Le ou les excipients avantageusement ajoutés lors des procédés ci-dessus sont utiles comme solution secondaire de dilution afin de pouvoir réaliser un traitement adaptatif aux différentes conditions cliniques rencontrées. Il s'agit d'eau purifiée osmosée (isotonique). Cet excipient sera préférentiellement identique à celui utilisé pour chacun des composés et dérivés mélangés et, s'il ne l'est pas, il sera compatible pharmaceutiquement pour pouvoir être mélanger ensemble, avant tout usage clinique. Cet excipient pourra en outre contenir des agents divers, pharmaceutiquement compatibles, avec l'ensemble des molécules du mélange thérapeutique final dans le but de modifier certaines propriétés physico-chimiques de la composition (exemples: les propriétés tensioactives, oxydantes, olfactives ou gustatives, la stabilité, le pH, le pKa, la densité, la solubilité, la viscosité, la coloration, le coefficient de partage eau-ectanol) par l'ajout de(s) l'agent(s) adéquat(s).

Cet excipient peut contenir des anti-oxydants et/ou des acides aminés qui auront un effet de dilution en neutralisant les oxydants de la solution active principale et tout particulièrement l'hypochlorite de métal alcalin. Ces anti-oxydants, ces acides aminés et leurs dérivés halogénés auront une action pharmaceutique soit neutre, soit dirigée vers les effets thérapeutiques recherchés, tout en ayant une toxicité moindre que les oxydants de la solution active principale. Dans tous les cas, ils devront être compatibles, pharmaceutiquement, avec les composés et dérivés mis en oeuvre dans ces procédés.

Le mélange selon l'invention peut se présenter sous toute forme adaptée à une application locale, comme un gel ou encore un aérosol.

L'invention s'intéresse tout particulièrement au traitement par voie locale des infections dues aux virus de la famille de l'herpesviridiae.

Le mélange de l'invention est avantageusement utilisée localement afin d'éviter des effets secondaires, comme l'augmentation du risque d'athérosclérose. Elle peut être appliquée sur toutes les muqueuses externes ou internes, buccales, génitales, vaginales, ophtalmiques, otiques, sinusales, rhinogènes, dermiques, etc. Le mélange de l'invention peut se présenter sous toute forme adaptée à cette administration et de préférence sous une forme semi-liquide, de préférence un gel grâce à l'addition d'une ou plusieurs substances pharmaceutiquement compatibles, comme la cellulose, ou encore des acides aminés, des peptides et/ou des protéines.

La composition de l'invention peut aussi être adaptée aux conditions cliniques et/ou aux muqueuses malades. Cette adaptation s'effectue en modifiant les concentrations en produits actifs des solutions thérapeutiques.

Selon une forme de réalisation particulière de l'invention, une addition d'antioxydants piégeant spécifiquement NaOH est conseillée.

Le mélange selon l'invention est utile pour le traitement local des maladies ou des processus inflammatoires chroniques et/ou progressifs et/ou aiguë. Elle est également indiquée pour le rinçage pré et/ou per et/ou post chirurgical des muqueuses internes et /ou externes et les plaies ouvertes. L'invention concerne tout particulièrement une méthode de traitement des lésions et affections décrites précédemment consistant en une mise en contact de la muqueuse à traiter avec la composition de la présente invention, par exemple (ENL) pendant une durée d'environ 20 à 60 secondes, avec une posologie de 2 à 3 applications par jour et non suivies d'un rinçage. La quantité de composition employée devra être suffisante afin que les agents actifs thérapeutiques ne soient pas tous neutralisés d'une manière ou d'une autre. La mise en contact ne devra pas rester statique. Les concentrations de la solution devront être adaptées à l'évolution de la situation clinique jusqu'à la guérison de la maladie.

Ainsi, l'invention concerne tout particulièrement le traitement local des lésions et infections liées aux parodontites chroniques et/ou aiguës. Ainsi, la composition de l'invention est remarquable pour être utilisée en irrigation du fond des poches parodontales dans le but d'éradiquer ces poches parodontales grâce à ses actions antiseptique, anti-inflammatoire, modulatrice de l'immunité et stimulatrice de la cicatrisation sur les tissus parodontaux (os alvéolaire, ligament alvéolo-dentaire et gencive).

La parodontite chronique est une maladie qui est due principalement à l'action pathogène de bactéries anaérobies, et tout particulièrement *Actinobacillus actinomycetemcomitans, Porphyromonas gingivalis, Bacteroides forsythus* et *Prevotella intermedia.* Ces bactéries provoquent un processus inflammatoire chronique qui a pour conséquence la destruction progressive du parodonte (tissu de soutien des dents). Le terme de cette maladie est la perte de la dent (odonte) suite à la disparition du tissu osseux de soutient.

Quelle que soit la phase du traitement de la parodontite chronique, l'irrigation des poches parodontales doit se faire en présence d'une puissante aspiration afin que la solution thérapeutique ne soit pas avalée ou inhalée par le patient. En cas de doute, ne pas hésiter à rincer abondamment.
i) Traitement d'attaque: Deux à trois semaines jusqu'à la disparition du saignement au sondage des poches parodontales (example de référence):
   - J 1 : après une rapide évaluation de la situation clinique, une irrigation de toutes les espaces créviculaires (qu'il y ait ou pas de poches parodontales) des dents de la cavité buccale sera effectuée. Un bain de bouche à base d'un mélange de chlorhexidine (composé de référence) (0,1 % du volume thérapeutique) et d'eau oxygénée (0,3 %) sera prescrit. La posologie sera d'un bain de bouche deux fois par jour (à distance du brossage des dents) pendant dix jours puis de une fois tous les deux à trois jours ad vitam aeterman (le traitement initial d'attaque devra être repris en cas d'halitose). Il sera fixé un planning de 2 à 3 rendez-vous par semaine.
   - Pour les autres séances, il sera procédé dans l'ordre : Enseignement, vérification et motivation de l'hygiène parodontale ; irrigation minutieuse (1 ml minimum de la solution par poche); Détartrage - surfaçage minutieux des racines dentaires.
   - Quand toutes les surfaces radiculaires seront propres et lisses, une séance devra être consacrée, après irrigation, au sondage des poches parodontales pour évaluer le degré de la maladie, et éventuellement d'autres examens, par exemple biologique, pourront être effectués.
ii) Traitement curatif primaire (Quatre semaines).
   - Irrigation minutieuse des poches parodontales tous les dix jours.
   - A la dernière séance du traitement curatif primaire, l'irrigation sera suivie d'un sondage puis d'un surfaçage des surfaces radiculaires.
iii) Traitement curatif secondaire (jusqu'à la disparition clinique des poches parodontale).
   - Irrigation minutieuse des poches parodontales tous les dix jours.
   - Toutes les trois séances du traitement curatif primaire, l'irrigation sera suivie d'un sondage puis d'un surfaçage des surfaces radiculaires.
iv) Traitement d'entretien.
   Même après avoir diagnostiqué une guérison clinique, le traitement devra se maintenir mais l'espace entre deux rendez-vous passera de deux à trois semaines, tout en respectant les autres caractéristiques du protocole opératoire du traitement curatif secondaire.
   Si au bout de deux mois, on ne constate pas de récidive mais plutôt une consolidation de la cicatrisation, on passera à la dernière phase, la surveillance.
   En présence de récidives, en fonction des conditions cliniques, on reprendra le traitement soit au niveau du traitement d'attaque, soit au niveau du traitement curatif primaire ou secondaire.
v) Surveillance.
   On fixera un rendez-vous toutes les six semaines. Il sera pratiqué un sondage minutieux avec recherche d'éventuelles récidives.
   - En absence de récidive, on pratiquera une irrigation de tous les espaces créviculaires suivie d'un surfaçage soigneux des surfaces radiculaires.
   - En présence de récidives, en fonction des conditions cliniques, on reprendra le traitement soit au niveau du traitement d'attaque, soit au niveau du traitement curatif.

   Dans cette application particulière, l'invention envisage aussi les traitements parodontaux chirurgicaux avec comblement osseux dans lesquels le ou les biomatériaux utilisés de comblement sont associés à la composition de l'invention et/ou à l'un de ces constituants.

### RÉFÉRENCES BIBLIOGRAPHIQUES

1. Bloomfield SF, Miles GA. - The antibacterial properties of sodium dichloroisocyanurate and sodium hypochlorite formulations. - J Appl Bacteriol. 1979 Feb;46(1):65-73.
2. Cantin AM. - Taurine modulation of hypochlorous acid-induced lung epithelial cell injury in vitro. Role of anion transport. - J Clin Invest. 1994 Feb;93(2):606-14.
3. Davies JM, Horwitz DA, Davies KJ. - Inhibition of collagenase activity by N-chlorotaurine, a product of activated neutrophils. - Arthritis Rheum. 1994 Mar;37(3):424-7.
4. Grisham MB, Jefferson MM, Melton DF, Thomas EL. - Chlorination of endogenous amines by isolated neutrophils. Ammonia-dependent bactericidal, cytotoxic, and cytolytic activities of the chloramines. - J Biol Chem. 1984 Aug 25;259(16):10404-13.
5. Hand RE, Smith ML, Harrison JW. - Analysis of the effect of dilution on the necrotic tissue dissolution property of sodium hypochlorite. - J Endod. 1978 Feb;4(2):60-4.
6. Heggers JP, Sazy JA, Stenberg BD, Strock LL, McCauley RL, Herndon DN, Robson MC. - Bactericidal and wound-healing properties of sodium hypochlorite solutions: the 1991 Lindberg Award. - J Burn Care Rehabil. 1991 Sep-Oct;12(5):420-4.
7. Hidalgo E, Dominguez C. - Growth-altering effects of sodium hypochlorite in cultured human dermal fibroblasts. - Life Sci. 2000 Aug 4;67(11):1331-44.
8. Huxtable RJ - Sources and turnover rates of taurine in nursing and weaned rat pups. - J. Nutr. 1981; 111 :1275-86
9. Kim C, Chung J-K, Jeong JM, Chang YS, Lee YJ, Kim YJ, Lee MC, Koh C-s, Kim B-K - Uptake of taurine and taurine chloramine in murine macrophages and their distribution in mice with experimental inflammation. - Adv Exp Med Biol. 1998; 442:169-76
10. Kim C, Park E, Quinn MR, Schuller-Levis G. - The production of superoxide anion and nitric oxide by cultured murine leukocytes and the accumulation of TNF-alpha in the conditioned media is inhibited by taurine chloramine. - Immunopharmacology. 1996 Sep;34(2-3):89-95
11. Knight JA. - Review: free radicals, antioxidants, and the immune system. - Annals of Clinical laboratory Science. 2000; 30(2) :145-58.
12. Kontny E, Grabowska A, Kowalczewski J, Kurowska M, Janicka I, Marcinkiewicz J, Maslinski W. - Taurine chloramine inhibition of cell proliferation and cytokine production by rheumatoid arthritis fibroblast-like synoviocytes. - Arthritis Rheum. 1999 Dec;42(12):2552-60.
13. Kontny E, Szczepanska K, Kowalczewski J, Kurowska M, Janicka I, Marcinkiewicz J, Maslinski W. - The mechanism of taurine chloramine inhibition of cytokine (interleukin-6, interleukin-8) production by rheumatoid arthritis fibroblast-like synoviocytes. - Arthritis Rheum. 2000 Oct ;43(10) :2169-77.
14. Kunsch C, Medford RM. - Oxidative stress as a regulator of gene expression in the vasculature. - Circ Res. 1999 Oct 15;85(8):753-66.
15. Lelianov AD, Grachev AM, Sergienko VI, Farashchuk NF, Kiriushenkova SV. - [The use of an electrolytic solution of sodium hypochlorite in acute suppurative diseases of the soft tissues]. - Klin Khir. 1991;(12):16-9. Russian.
16. Liu Y, Schuller-Levis G, Quinn MR. - Monocyte chemoattractant protein-1 and macrophage inflammatory protein-2 production is inhibited by taurine chloramine in rat C6 glioma cells. - Immunol Lett. 1999 Oct 1;70(1):9-14.
17. Liu Y et al. - Taurine chloramine inhibits production of nitric oxide and protaglandin E2 in activated C6 glioma cells by suppressing inducible nitric oxide synthase and cyclooxygenase-2 expression. - Brain res mol brain res 1998 Aug 31 ;59(2) :189-95.
18. Male D., Champion B., Cooke A. - Advanced immunology. - 2^{nd} ed. philadelphia: J.B. Lippincott company ; 1989 ;5 :1-15
19. Marquez LA, Dunford HB. - Chlorination of taurine by myeloperoxidase. Kinetic evidence for an enzyme-bound intermediate. - J Biol Chem. 1994 Mar 18;269(11):7950-6.
20. Marcinkiewicz J, Czajkowska B, Grabowska A, Kasprowicz A, Kociszewska B. - Differential effects of chlorination of bacteria on their capacity to generate NO, TNF-alpha and IL-6 in macrophages. - Immunology. 1994 Dec;83(4):611-6
21. Marcinkiewicz J et Al. - Taurine chloramine, a product of actived neutrophils, inhibits in vitro the génération of nitric oxide and other macrophage inflammatory mediators. - Journal of leukocyte biology 1995 Dec ;58: 667-74
22. Marcinkiewicz J. - Regulation of cytokine production by eicosanoids and nitric oxide. - Arch Immunol Ther Exp (Warsz). 1997;45(2-3):163-7
23. Marcinkiewicz J. - Nitric oxide and antimicrobial activity of reactive oxygen intermediates. - Immunopharmacology. 1997 Aug;37(1):35-41
24. Marcinkiewicz J. - Neutrophil chloramines: missing links between innate and acquired immunity. - Immunol Today. 1997 Dec;18(12):577-80.
25. Marcinkiewicz J, Grabowska A, Bereta J, Bryniarski K, Nowak B. - Taurine chloramine down-regulates the generation of murine neutrophil inflammatory mediators. - Immunopharmacology. 1998 Jul;40(l):27-38.
26. Marcinkiewicz J, Grabowska A, Chain BM. - Modulation of antigen-specific T-cell activation in vitro by taurine chloramine. - Immunology. 1998 Jul;94(3):325-30.
27. Marcinkiewicz J, Nowak B, Grabowska A, Bobek M, Petrovska L, Chain B. - Regulation of murine dendritic cell functions in vitro by taurine chloramine, a major product of the neutrophil myeloperoxidase-halide system. - Immunology. 1999 Nov;98(3):371-8.
28. Marcinkiewicz J, Chain B, Nowak B, Grabowska A, Bryniarski K, Baran J. - Antimicrobial and cytotoxic activity of hypochlorous acid: interactions with taurine and nitrite. - Inflamm Res. 2000 Jun;49(6):280-9.
29. Megarbane B, Galanaud P, Emilie D - Cytokines du système de défense: interleukines et chimiokines - Médecine Thérapeutique. 1998 Oct; 4(8) : 641-53.
30. Moeslinger T, Friedl R, Volf I, Brunner M, Koller E, Spieckermann PG. - Inhibition of inducible nitric oxide synthesis by oxidized lipoprotein(a) in a murine macrophage cell line. - FEBS Lett. 2000 Jul 28;478(1-2):95-9.
31. Owen WF Jr, Soberman RJ, Yoshimoto T, Sheffer AL, Lewis RA, Austen KF. - Synthesis and release of leukotriene C4 by human eosinophils. J Immunol. - 1987 Jan 15;138(2):532-8.
32. Park E, Schuller-Levis G, Jia JH, Quinn MR. - Preactivation exposure of RAW 264.7 cells to taurine chloramine attenuates subsequent production of nitric oxide and expression of iNOS mRNA. - J Leukoc Biol. 1997 Feb;61(2):161-6.
33. Park E, Quinn MR, Wright CE, Schuller-Levis G. - Taurine chloramine inhibits the synthesis of nitric oxide and the release of tumor necrosis factor in activated RAW 264.7 cells. - J Leukoc Biol. 1993 Aug;54(2):119-24.
34. Pullar JM, Winterbourn CC, Vissers MC. - Loss of GSH and thiol enzymes in endothelial cells exposed to sublethal concentrations of hypochlorous acid. - Am J Physiol. 1999 Oct;277(4 Pt 2):H1505-12.
35. Quinn MR, Park E, Schuller-Levis G. - Taurine chloramine inhibits prostaglandin E2 production in activated RAW 264.7 cells by post-transcriptional effects on inducible cyclooxygenase expression. - Immunol Lett. 1996 May;50(3):185-8.
36. Segura JJ, Jimenez-Rubio A, Guerrero JM, Calvo JR. - Comparative effects of two endodontic irrigants, chlorhexidine digluconate and sodium hypochlorite, on macrophage adhesion to plastic surfaces. - J Endod. 1999 Apr;25(4):243-6.
37. Sen CK, Packer L. - Antioxidant and redox regulation of gene transcription. - FASEB J. 1996 May ; 10(7): 709-20.
38. Shih M, Marshall FJ, Rosen S. - The bactericidal efficiency of sodium hypochlorite as an endodontic irrigant. - Oral Surg Oral Med Oral Pathol. 1970 Apr;29(4):613-9.
39. Tatsumi T, Fliss H. - Hypochlorous acid and chloramines increase endothelial permeability: possible involvement of cellular zinc. - Am J Physiol. 1994 Oct; 267 (4 Pt 2) :H1597-607.
40. The SD. - The solvent action of sodium hypochlorite on fixed and unfixed necrotic tissue. - Oral Surg Oral Med Oral Pathol. 1979 Jun;47(6):558-61.
41. Vissers MC, Pullar JM, Hampton MB. - Hypochlorous acid causes caspase activation and apoptosis or growth arrest in human endothelial cells. - Biochem J. 1999 Dec 1;344 Pt 2:443-9.
42. Vile GF, Rothwell LA, Kettle AJ. - Initiation of rapid, P53-dependent growth arrest in cultured human skin fibroblasts by reactive chlorine species. - Arch Biochem Biophys. 2000 May 1;377(1):122-8.
43. Vogt W. - Complement activation by myeloperoxidase products released from stimulated human polymorphonuclear leukocytes. - Immunobiology. 1996 Aug; 195(3):334-46.
44. Wright CE, Tallan HH, Lin YY, Gaull GE. - Taurine: biological update. - Annu Rev Biochem. 1986;55:427-53.
45. Zgliczynski JM, Stelmaszynska T, Domanski J, Ostrowski W. - Chloramines as intermediates of oxidation reaction of amino acids by myeloperoxidase. - Biochim Biophys Acta. 1971 Jun 16;235(3):419-24

## Revendications

1. Utilisation du mélange d'au moins un hypochlorite de métal alcalin et d'au moins une N-haloamine taurine pour la préparation d'un médicament destiné, chez l'homme ou l'animal, au traitement et/ou à la prévention d'infections virales et/ou bactériennes et/ou parasitaires et/ou fongiques et/ou par des agents transmissibles non conventionnels (ATNC) ledit médicament n'exerçant pas de stimulation de l'activité de la myéloperoxydase.

2. Utilisation du mélange d'au moins un hypochlorite de métal alcalin et d'au moins une N-haloamine taurine pour la préparation d'un médicament destiné, chez l'homme ou l'animal, au traitement d'inflammations chroniques, progressives ou aiguës, ledit médicament n'exerçant pas de stimulation de l'activité de la myéloperoxydase.

3. Utilisation du mélange d'au moins un hypochlorite de métal alcalin et d'au moins une N-haloamine taurine pour la préparation d'un médicament destiné, chez l'homme ou l'animal, à un traitement immuno-modulateur, ledit médicament n'exerçant pas de stimulation de l'activité de la myéloperoxydase.

4. Utilisation du mélange d'au moins un hypochlorite de métal alcalin et d'au moins une N-haloamine taurine pour la préparation d'un médicament destiné, chez l'homme ou l'animal, à une stimulation de la cicatrisation tissulaire, ledit médicament n'exerçant pas de stimulation de l'activité de la myéloperoxydase.

5. Utilisation du mélange d'au moins un hypochlorite de métal alcalin et d'au moins une N-haloamine taurine pour la préparation d'un médicament destiné, chez l'homme ou l'animal, aux rinçages pré et/ou per et/ou post chirurgicaux, ledit médicament n'exerçant pas de stimulation de l'activité de la myéloperoxydase.

6. Utilisation selon l'une des revendications 1 à 5, **caractérisée en ce que** le médicament est utile pour le traitement local des lésions et infections liées aux parodontites chroniques et/ou aiguës.

7. Utilisation selon l'une des revendications 1 à 6, **caractérisée en** en ce que le médicament est utile pour le traitement local des lésions et infections liées aux virus de la famille des herpesviridiae.

8. Utilisation selon l'une quelconque 1 à 7, **caractérisé en ce que** l'hypochlorite de métal alcalin est l'hypochlorite de sodium et **en ce que** la N-haloamine taurine est la N-chloramine taurine.

## Claims

1. Use of a mixture of:
- (i) at least one hypochlorite of alkaline metal, and
- (ii) at least one taurine N-haloamine,
for the preparation of a drug aimed to the treatment and/or to the prevention of viral infections, and/or bacterial infections, and/or parasitical infections and/or fungal infections and/or diseases generated from non conventional transmissible agents, in humans or animals; said drug does not generate a stimulation of myeloperoxidase activity.

2. Use of a mixture of:
- (i) at least one hypochlorite of alkaline metal, and
- (ii) at least one taurine N-haloamine,
for the preparation of a drug aimed to the treatments of chronic inflammation, and/or progressive inflammation, and/or acute inflammation, in humans or animals; said drug does not generate a stimulation of myeloperoxidase activity.

3. Use of a mixture of:
- (i) at least one hypochlorite of alkaline metal, and
- (ii) at least one taurine N-haloamine,
for the preparation of a drug aimed to immunomodulating treatments, in humans or animals; said drug does not generate a stimulation of myeloperoxidase activity.

4. Use of a mixture of:
- (i) at least one hypochlorite of alkaline metal, and
- (ii) at least one taurine N-haloamine,
for the preparation of a drug aimed to the stimulation of the tissue healing, in humans or animals; said drug does not generate a stimulation of myeloperoxidase activity.

5. Use of a mixture of:
- (i) at least one hypochlorite of alkaline metal, and
- (ii) at least one taurine N-haloamine,
for the preparation of a drug aimed to the pre-surgical irrigation, and/or the per-surgical irrigation, and/or the post-surgical irrigation, in humans or animals; said drug does not generate a stimulation of myeloperoxidase activity.

6. Use according to any one of claims 1 to 5, wherein said drug is useful for the local treatment of lesions and infections linked to chronic and/or acute periodontitis.

7. Use according to any one of claims 1 to 6, wherein said drug is useful for the local treatment of lesions and infections linked to the virus of the herpesviridiae family.

8. Use according to any one of claims 1 to 7, wherein the hypochlorite of alkaline metal is the sodium hypochlorite, and the taurine N-haloamine is the taurine N-chloramine.

## Patentansprüche

1. Anwendung der Mischung wenigstens eines alkalischen Metall Hypochlorits und wenigstens eines N-haloamin Taurins zur Vorbereitung eines Artzneimittels bestimmt, beim Menschen und beim Tier, zur Behandlung und/oder zur Vorbeugung von viralen und/oder bakteriellen und/oder parasitären und/oder pilzartigen und/oder durch nicht konventionelle übertragbare Erreger(ATNC) hervorgerufene Infektionen, besagtes Artzneimittel nicht ausübend eine Stimulierung der Aktivität der Myeloperoxydase.

2. Anwendung der Mischung wenigstens eines alkalischen Metall Hypochlorits und wenigstens eines N-haloamin Taurins zur Vorbereitung eines Artzneimittels bestimmt, beim Menschen und beim Tier, zur Behandlung von chronischen Entzündungen, progressiven oder akuten, besagtes Artzneimittel nicht ausübend eine Stimulierung der Aktivität der Myeloperoxydase.

3. Anwendung der Mischung wenigstens eines alkalischen Metall Hypochlorits und wenigstens eines N-haloamin Taurins zur Vorbereitung eines Artzneimittels bestimmt, beim Menschen und beim Tier, zu einer immunomodulierenden Behandlung, besagtes Artzneimittel nicht ausübend eine Stimulierung der Aktivität der Myeloperoxydase.

4. Anwendung der Mischung wenigstens eines alkalischen Metall Hypochlorits und wenigstens eines N-haloamin Taurins zur Vorbereitung eines Artzneimittels bestimmt, beim Menschen und beim Tier, zu einer Stimulierung der Gewebevernarbung, besagtes Artzneimittel nicht ausübend eine Stimulierung der Aktivität der Myeloperoxydase.

5. Anwendung der Mischung wenigstens eines alkalischen Metall Hypochlorits und wenigstens eines N-haloamin Taurins zur Vorbereitung eines Artzneimittels bestimmt, beim Menschen und beim Tier, zur vor und/oder per und/oder post chirurgischen Spülung, besagtes Artzneimittel nicht ausübend eine Stimulierung der Aktivität der Myeloperoxydase.

6. Anwendung entsprechend einem der Ansprüche 1 bis 5, charakterisiert **dadurch** dass das Artzneimittel nützlich ist für die lokale Behandlung der Wunden und Infektionen verbunden mit chronischen und/oder akuten Paradontiten.

7. Anwendung entsprechend einem der Ansprüche 1 bis 6, charakterisiert **dadurch** dass das Artzneimittel nützlich ist für die lokale Behandlung der Wunden und Infektionen verbunden mit Viren der Familie der Herpesviridiae.

8. Anwendung entsprechend einem der Ansprüche 1 bis 7, charakterisiert **dadurch** dass das alkalische Metall Hypochlorit Natrium Hypochlorit ist und **dadurch** dass das N-haloamin Taurin N-chloramin Taurin ist.
